(19) Europäisches Patentamt / European Patent Office / Office européen des brevets

(11) **EP 4 019 622 A1**

(12) **DEMANDE DE BREVET EUROPEEN**

(43) Date de publication:
**29.06.2022   Bulletin 2022/26**

(21) Numéro de dépôt: **21217333.0**

(22) Date de dépôt: **23.12.2021**

(51) Classification Internationale des Brevets (IPC):
**C12M 1/36** *(2006.01)*     **C12M 1/09** *(2006.01)*
**C12M 1/34** *(2006.01)*     **G01N 33/14** *(2006.01)*
**G01N 7/20** *(2006.01)*

(52) Classification Coopérative des Brevets (CPC):
**C12M 41/48; C12M 23/56; C12M 41/12;
C12M 41/36; G01N 7/20; G01N 33/14**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(30) Priorité: **23.12.2020   FR 2014076**

(72) Inventeurs:
• **PORTE, Florian**
 **38054 GRENOBLE CEDEX 09 (FR)**
• **PERRIER, Régis**
 **38054 GRENOBLE CEDEX 09 (FR)**

(74) Mandataire: **Brevalex
56, Boulevard de l'Embouchure
B.P. 27519
31075 Toulouse Cedex 2 (FR)**

(71) Demandeur: **Commissariat à l'énergie atomique
et aux énergies alternatives
75015 Paris (FR)**

(54) **SYSTEME DE SURVEILLANCE D'UN PROCESSUS BIOCHIMIQUE**

(57)    L'invention porte sur un procédé et un système de surveillance in-situ d'un processus biochimique dans un réacteur comprenant une cuve (5) destinée à recevoir un liquide (7), ledit système comportant :
- un dispositif de mesure (9) destiné à être introduit en flottant dans ladite cuve (5), ledit dispositif de mesure (9) étant instrumenté de capteurs configurés pour réaliser à des instants successifs des mesures relatives au processus biochimique et pour transmettre auxdits instants successifs des données d'observation représentatives desdites mesures, et
- un dispositif de contrôle (11) configuré pour commander auxdits instants successifs la régulation du réacteur (3) biochimique en fonction desdites données d'observation reçues depuis le dispositif de mesure (9).

FIG.1

EP 4 019 622 A1

## Description

### Domaine technique

La présente invention concerne le domaine de surveillance de processus biochimique.

### Etat de la technique antérieure

La régulation d'un procédé biochimique peut adresser de nombreuses applications industrielles dans des domaines variés comme par exemple, les agroalimentaires (vin, cidre, bière, spiritueux, aliments liquides...), les pharmaceutiques (médicaments ou nutriments complémentaires...), les environnementaux (analyse de l'eau dans des stations d'épuration, méthanisation...), les pétrochimiques (biogaz...), les électrochimiques etc.

Les processus biochimiques impliquent l'action de microorganismes sur des substrats complexes (jus de fruits, lixiviats, ...). Leur régulation en milieu industriel comme pour l'agroalimentaire est en général faite par des analyses manuelles hors ligne « off-line ». La régulation dans des bioréacteurs par un suivi de paramètres de contrôles en ligne « on-line » est réservée aux produits de haute valeur ajoutée comme les produits médicaux.

Les paramètres de contrôles comportent en général des mesures de température, et éventuellement d'autres mesures complémentaires qui peuvent être spécifiques à l'application comme par exemple l'acidité, la turbidité, le dégagement gazeux, la densité, l'oxygène dissout, etc.

Dans le cas des boissons alcooliques, on a besoin de mesurer à minima la température et la densité d'un liquide pour contrôler la qualité alimentaire comme le taux de sucre et d'alcool. Pour réaliser des médicaments ou pour analyser l'eau dans des stations d'épurations, les mesures concernent plus généralement le pH, le potentiel Redox, la conductivité et la présence d'oxygène et de gaz carbonique dissout etc.

Concernant, la mesure de densité, il existe des densimètres manuels qui sont couramment répandus dans divers domaines d'activités. Leurs dimensions et leur poids définissent la gamme et la précision de mesure. Toutefois, ils nécessitent des prélèvements pour faire des analyses hors ligne et les opérations sont totalement manuelles.

Il existe des densimètres électroniques qui mesurent la fréquence d'oscillation de tubes en forme de U qui varient selon la masse du liquide prélevé. Ces appareils peuvent être utilisés en ligne mais sont chers et sensibles à certaines perturbations (encrassement, bulles).

Il y a par ailleurs, des appareils qui déterminent la densité d'un liquide en mesurant l'indice de réfraction. Toutefois, la mesure peut être perturbée par la présence de gaz (par exemple, $CO_2$) ou l'encrassement du capteur.

Tous ces appareils permettent dans certains cas des mesures en ligne « on line » mais pas « in situ ». Il existe cependant, des modules autonomes en forme de boule destinés à mesurer le pH, la conductivité, la pression et le mouvement dans un liquide. Ces modules peuvent être en libre circulation dans des bioréacteurs et permettent ainsi, des mesures in situ. Toutefois, ces modules sont limités à des mesures très spécifiques et ne sont pas adaptés pour mesurer la densité. En outre, la transmission des données n'est pas aisée depuis le milieu liquide et de plus, on ignore leur localisation dans le liquide engendrant un manque de référence spatiale des mesures.

Par ailleurs les bioprocédés impliquent en général la régulation de la température, de l'agitation, de l'oxygénation et de l'apport de nutriments. La détermination de ces paramètres de contrôles a pour but d'améliorer à la fois la qualité et le rendement du produit tout en optimisant les apports en énergie et en nutriments. Ce contrôle repose généralement sur le savoir-faire de l'utilisateur (œnologue dans le cas du vin, microbiologiste pour les produits pharmaceutiques...) et peut ainsi être très fastidieux tout en prenant beaucoup de temps.

L'objet de la présente invention est de remédier aux inconvénients précités en proposant un ajustement automatique et précis des paramètres de contrôle du produit recherché en utilisant une surveillance en temps réel et in situ de l'évolution du processus biochimique tout en permettant des mesures multiparamétriques précises au moyen d'un seul appareil de mesure.

### Présentation de l'invention

L'invention a pour objet un système de surveillance in-situ d'un processus biochimique dans un réacteur comprenant une cuve destinée à recevoir un liquide, ledit système comportant :

- un dispositif de mesure destiné à être introduit dans ladite cuve, ledit dispositif de mesure étant instrumenté de capteurs configurés pour réaliser à des instants successifs des mesures relatives au processus biochimique et pour transmettre auxdits instants successifs des données d'observation représentatives d'au moins la température et la densité, et
- un dispositif de contrôle configuré pour commander à des instants successifs la régulation du réacteur biochimique en fonction desdites données d'observation reçues depuis le dispositif de mesure.

Ainsi, le dispositif de mesure permet de suivre l'évolution des paramètres physiques et de les transmettre au dispositif de contrôle de sorte que ce dernier puisse commander par rétroaction et en temps réel la réaction dans la cuve. Ce système permet ainsi de contrôler automatiquement le processus biochimique tout en réagissant directement en temps réel en cas de détection d'une anomalie dans le processus.

Avantageusement, lesdites mesures comportent des mesures de température et de densité du liquide et au moins un autre type de mesures parmi les mesures suivantes : mesures mécaniques de pressions et/ou de débit gazeux et/ou d'accélérations et/ou de niveau de flottaison, mesures électriques de tensions et/ou de courants et/ou de fréquences de résonance, et mesures optiques.

Ceci permet d'avoir plusieurs indicateurs pour une rétroaction et un contrôle plus efficace et plus précis du processus biochimique.

Selon un mode de réalisation, le dispositif de mesure comporte un microprocesseur configuré pour déterminer auxdits instants successifs des vecteurs de variables physiques relatives au processus biochimique en fonction des mesures correspondantes réalisées auxdits instants successifs, les données d'observation transmises par le dispositif de mesure audit dispositif de contrôle comportant lesdits vecteurs de variables physiques et lesdites mesures correspondantes.

Ainsi, le dispositif de mesure traite les mesures pour déterminer au cours du temps des vecteurs de valeurs physiques d'intérêt dont la densité avant de les transmettre au dispositif de contrôle.

Selon un autre mode de réalisation les données d'observation transmises par le dispositif de mesure audit dispositif de contrôle comportent lesdites mesures. Dans ce cas, le dispositif de contrôle est configuré pour déterminer auxdits instants successifs des vecteurs de variables physiques relatives au processus biochimique en fonction des mesures correspondantes.

Selon ce mode de réalisation, le dispositif de mesure transmet uniquement les mesures et c'est le dispositif de contrôle qui les traite pour déterminer les paramètres physiques d'intérêt. Ceci permet de minimiser le traitement et la consommation électrique au niveau du dispositif de mesure .

Chacun desdits vecteurs de variables physiques comporte une variable de température, une variable de densité du liquide déterminée à partir des mesures de niveaux de flottaison et/ou de pressions, et au moins une autre variable parmi les variables suivantes : dégagement gazeux déterminé à partir des mesures de pressions,, conductivité et/ou permittivité électriques du liquide déterminées à partir des mesures électriques, mouvement du liquide déterminé à partir des mesures d'accélération, PH et/ou potentiel redox déterminés à partir des mesures électriques, oxygène et/ou $CO_2$ dissout déterminés à partir des mesures électriques et/ou mesures optiques, spectre d'absorption optique et/ou pouvoir rotatoire déterminés à partir des mesures optiques.

Ces indicateurs physiques permettent de détecter avec une grande précision la moindre anomalie dans le processus biochimique.

Avantageusement, le dispositif de contrôle est configuré pour commander la régulation du réacteur biochimique par au moins une action parmi les actions suivantes : modification de la vitesse d'agitation, modification de la température, modification du débit d'apport en oxygène, apport de nutriments ou autres éléments d'activation ou de stabilisation du processus biochimique, apport de levures ou de souches bactériennes.

Avantageusement, le dispositif de contrôle est configuré pour :

- prédire auxdits instants successifs des vecteurs prédictifs de variables physiques en fonction desdits vecteurs de variables physiques précédents issus des mesures,
- prédire auxdits instants successifs des mesures anticipatives en fonction desdits vecteurs prédictifs de variables physiques,
- calculer auxdits instants successifs des écarts de mesures entre les mesures anticipatives et les mesures réelles correspondantes,
- corriger auxdits instants successifs les vecteurs de variables physiques en fonction desdits écarts de mesures,
- déterminer auxdits instants successifs des vecteurs d'actions de régulation en fonction desdits vecteurs de variables physiques correspondants, et
- commander la régulation du réacteur biochimique en déclenchant auxdits instants successifs des actions de régulation basées sur lesdits vecteurs d'actions de régulation.

Ceci permet de déterminer avec précision et pour chaque instant de temps, une série d'actions en connaissance des variables physiques observables grâce aux capteurs du dispositif de mesure .

Avantageusement, le dispositif de contrôle est configuré pour déterminer les vecteurs d'actions de régulation en fonction des vecteurs prédictifs de variables physiques en cas de défaut du dispositif de mesure

Ceci permet de poursuivre la régulation du processus en cas d'un dysfonctionnent momentané du dispositif de mesure .

Avantageusement, le dispositif de contrôle est configuré pour :

- prédire chaque vecteur prédictif de variables physiques en utilisant une première fonction f temporelle définissant des valeurs de variables physiques en connaissance de leurs valeurs à un instant de temps précédent, ladite première fonction f étant prédéterminée par un modèle de Markov standard d'ordre d'au moins 1,
- prédire chaque mesure anticipative en utilisant une deuxième fonction g associant les mesures réalisées par le dispositif de mesure aux variables physiques à un instant de temps donné, ladite deuxième fonction g étant prédéterminée par des équations liant les mesures et les variables physiques, et
- détermination de chaque vecteur d'actions de régulation en utilisant une troisième fonction h définissant une correspondance entre les actions à réaliser et les valeurs des variables physiques à un instant de temps donné, ladite troisième fonction h étant prédéterminée par une sélection préalable de seuils de déclenchement d'actions.

Ces fonctions peuvent être préadaptées pour être utilisées de manière simple pour le contrôle de toute réaction biochimique.

Avantageusement, le système de surveillance comporte une base de données construite lors d'une phase d'apprentissage de régulation comprenant des données de correspondance entre des mesures $m_{1:T}$ réalisées par le dispositif de mesure et des actions $a_{1:T}$ réalisées dans la cuve, lesdites données de correspondance étant générées automatiquement par un processus d'apprentissage, et au moins l'une quelconque desdites première, deuxième et troisième fonctions est déterminée à partir desdites données de correspondance acquises depuis ladite base de données.

Ainsi, dans le cas où au moins une des trois fonctions est inconnue, elle peut être apprise automatiquement en connaissance d'une série temporelle complète de réaction biochimique réalisée précédemment par des experts du processus biochimique.

Avantageusement, le dispositif de contrôle est configuré en outre pour construire un modèle d'apprentissage de propriétés définissant des liens associant des propriétés p du produit final en fonction des variables physiques $\theta_{1:T}$ et caractéristiques c correspondantes. La connaissance du modèle d'apprentissage de propriétés permet d'obtenir un produit aux propriétés p, sachant ses caractéristiques c, par la régulation de ses variables physiques $\theta_{1:T}$ lors du processus de réaction biochimique. En effet, en connaissant d'une part le modèle qui relie les variables physiques et les caractéristiques aux propriétés du produit, et d'autre part les caractéristiques d'entrées c (par ex. dans le cas du vin ça pourrait être le cépage), le dispositif de contrôle peut lors de la réaction biochimique contraindre les variables physiques $\theta_{1:T}$ à suivre un certain profil pour obtenir des propriétés p désirées en sortie. Autrement dit, le dispositif de contrôle permet d'adapter les caractéristiques des éléments mis dans la cuve et de réguler l'évolution des variables physiques de réactions biochimiques selon les caractéristiques des éléments mis dans la cuve afin d'obtenir des propriétés spécifiques souhaitées du produit en sortie du réacteur.

Avantageusement, le dispositif de contrôle est configuré pour déterminer lesdits liens par estimation d'une fonction d'apprentissage au moyen d'un modèle statistique de type régression polynomiale, ou modèle à noyau, ou réseau de neurones.

Ainsi, la fonction d'apprentissage permet de comprendre l'influence des caractéristiques des éléments mis en réaction ou de l'évolution du processus biochimique dans les propriétés obtenues sur le produit.

Avantageusement, le dispositif de mesure comporte au moins une sonde de température, et un capteur de pression différentielle comprenant deux tubes reliés de longueur différentes.

Avantageusement, le dispositif de mesure est intégré dans un flotteur comportant :

- au moins une sonde de température,
- un capteur de pression différentielle comprenant deux tubes de longueur différentes en partie immergées dans le liquide,
- au moins deux capteurs électromagnétiques, électrochimiques ou optiques comprenant des parties sensibles fixées sur les parois du flotteur à proximité du liquide,
- un capteur de mouvement configuré pour mesurer les mouvements du flotteur,
- un module de communication sans fils comprenant une antenne disposée sur la partie haute du flotteur, le module de communication étant destiné à transmettre les données relatives aux mesures réalisées par les capteurs,
- un module de gestion de l'énergie comprenant un moyen d'alimentation électrique disposé dans la partie basse du flotteur, et
- un circuit électronique relié à tous les capteurs et éléments électroniques intégrés dans le flotteur, le circuit électronique comprenant un microprocesseur destiné à gérer l'acquisition de l'ensemble des capteurs, un premier niveau de traitement et la transmission des données.

Le flotteur est très peu encombrant et peut être installé de manière très simple dans une grande variété de cuves quelle que soit leur niveau de remplissage. De plus, il permet la mesure de densité de manière très précise à la fois dans un régime transitoire au début du processus ainsi que dans un régime permanent. En outre, l'antenne étant disposée dans la partie émergente du flotteur permet de transmettre les données de manière simple, régulière et précise.

Avantageusement, le flotteur comporte un module de calibration et de recharge inductive destiné à calibrer le flotteur et à télé-recharger le moyen d'alimentation intégrée dans le flotteur, ledit module de calibration et de recharge inductive étant compris dans un étui de protection du flotteur.

Ainsi, le flotteur est complétement autonome tout en permettant une fusion et une synthèse de mesures variées dans un unique dispositif embarqué.

L'invention vise également un procédé de surveillance in-situ d'un processus biochimique dans un réacteur comprenant une cuve destinée à recevoir un liquide, ledit procédé comportant les étapes suivantes :

- réaliser à des instants successifs des mesures relatives au processus biochimique,
- transmettre auxdits instants successifs des données d'observation représentatives d'au moins la température et la densité, et

- commander auxdits instants successifs la régulation du réacteur biochimique en fonction desdites données d'observation.

D'autres avantages et caractéristiques de l'invention apparaîtront dans la description détaillée non limitative ci-dessous.

**Brève description des figures**

On décrira à présent, à titre d'exemples non limitatifs, des modes de réalisation de l'invention, en se référant aux dessins annexés, dans lesquels :

[Fig. 1] illustre très schématiquement un système de surveillance in-situ d'un processus biochimique dans un réacteur biochimique, selon un mode de réalisation de l'invention ;
[Fig. 2A] et
[Fig. 2B] illustrent un algorithme de contrôle du réacteur biochimique, selon un mode de réalisation préféré de l'invention ;
[Fig. 3] est un graphe orienté représentant un algorithme d'apprentissage de propriété du composé à la sortie du réacteur biochimique, selon un mode de réalisation préféré de l'invention ;
[Fig. 4] illustre très schématiquement un système de surveillance in-situ d'un processus biochimique de fermentation dans un réacteur, selon un mode de réalisation de l'invention ;
[Fig. 5] illustre très schématiquement un procédé de surveillance in-situ du processus biochimique de fermentation en relation avec le système de la Fig. 4 ;
[Fig. 6] illustre très schématiquement un système de surveillance in-situ d'un processus biochimique dans un réacteur biochimique, selon un mode de réalisation préféré de l'invention ;
[Fig. 7] illustre très schématiquement un flotteur instrumenté, selon un mode de réalisation préféré de l'invention ; et
[Fig. 8] illustre de manière très schématique la disposition d'un capteur électromagnétique capacitif sur la surface latérale du flotteur, selon le mode de réalisation de la Fig. 7.

**Description des modes de réalisation**

Le principe de l'invention est de contrôler automatiquement un processus biochimique se déroulant dans une cuve en utilisant des mesures réalisées in situ.
Ainsi, l'invention propose d'ajuster les paramètres de contrôle en fonction des caractéristiques de la matière première et des propriétés du produit recherché tout au long du processus biochimique selon son évolution. En outre, ce contrôle est réalisé grâce à une surveillance en temps réel et in situ de l'évolution du processus biochimique tout en permettant des mesures multiparamétriques précises au moyen d'un seul appareil de mesure.

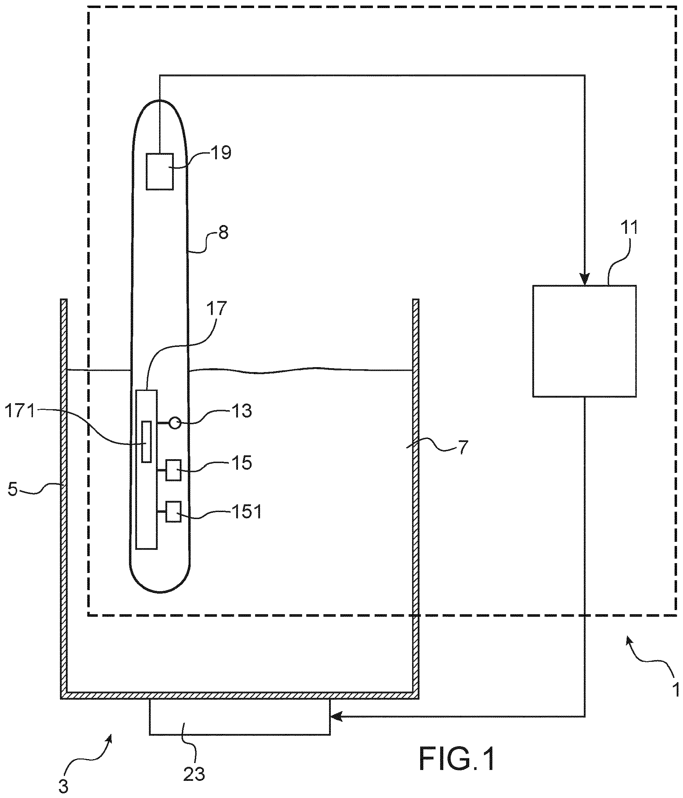

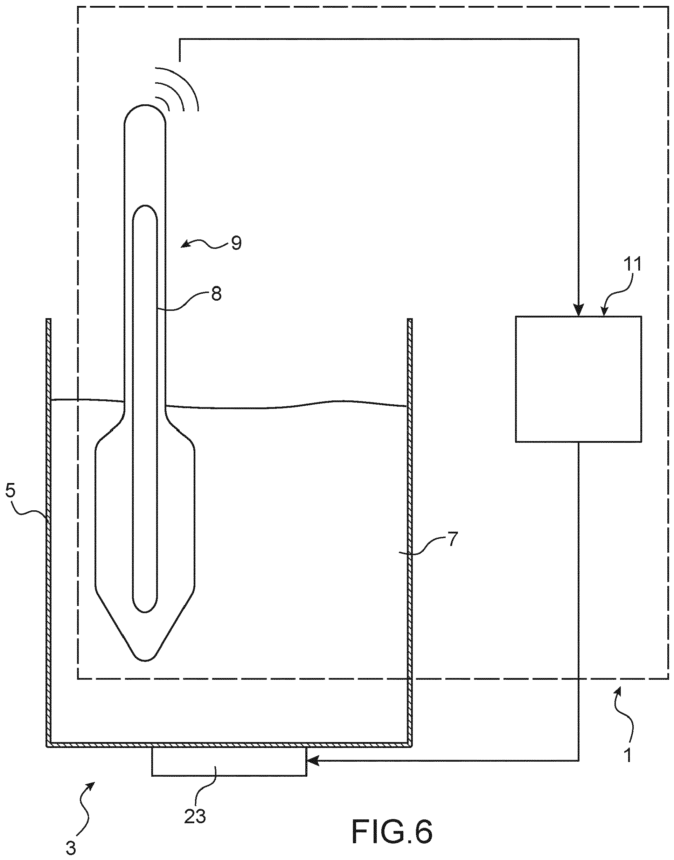

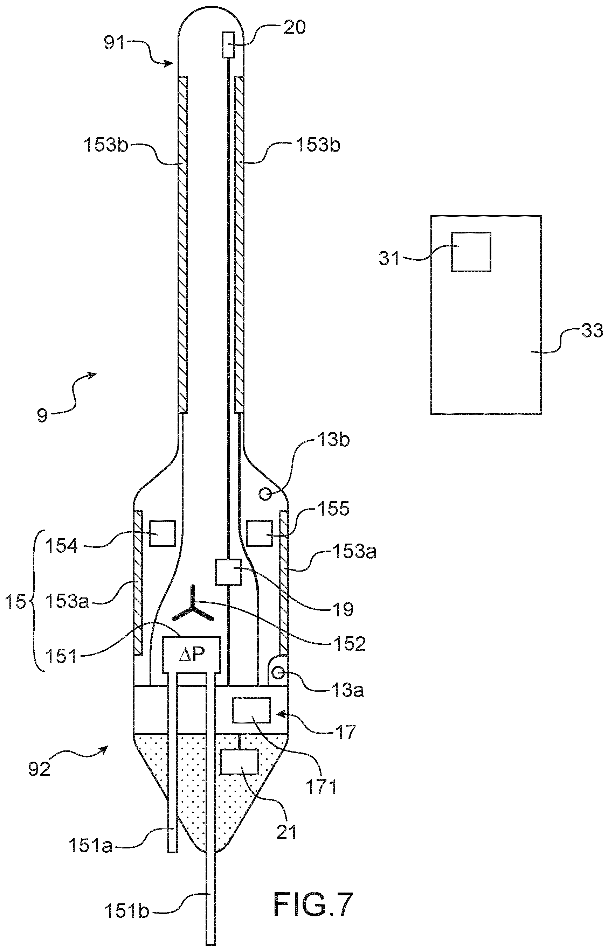

La Fig. 1 illustre très schématiquement un système 1 de surveillance in-situ d'un processus biochimique dans un réacteur 3 biochimique, selon un mode de réalisation de l'invention.
Le réacteur 3 biochimique comporte une cuve 5 destinée à recevoir un substrat liquide 7 sur lequel doit être réalisé un processus biochimique impliquant l'action de microorganismes. Par ailleurs, le système 1 de surveillance comporte un dispositif de mesure 8 et un dispositif de contrôle 11.
Selon un mode de réalisation, le dispositif de mesure 8 peut être fixé par exemple, par le haut de la cuve 5 tout en étant partiellement immergé dans le liquide. Ce mode de réalisation est avantageux dans le cas des petits réacteurs.
Selon un autre mode de réalisation, le dispositif de mesure 8 peut être embarqué ou intégré dans un dispositif flottant destiné à être introduit en flottant de manière partiellement immergée dans le liquide 7 contenu dans la cuve 5 (voir Figs. 6 et 7). Ce mode de réalisation est avantageux pour tout type de réacteurs et en particulier, dans le cas des grandes cuves avec un niveau de remplissage variable.
Le dispositif de mesure 8 est instrumenté de capteurs de mesures configurés pour réaliser directement sur le liquide 7 et à des instants successifs des mesures relatives au processus biochimique pour transmettre aux instants successifs des données d'observation représentatives de ces mesures au dispositif de contrôle 11.
Le dispositif de contrôle 11 (par exemple un ordinateur ou un microordinateur) est configuré pour recevoir les données d'observation transmises par le dispositif de mesure 8 et pour commander aux instants successifs la régulation du réacteur 3 biochimique en fonction des données d'observation reçues depuis le dispositif de mesure 8.
Le système 1 de surveillance permet ainsi de contrôler automatiquement le processus biochimique tout en réagissant directement en temps réel en cas de détection d'une anomalie dans le processus.
Avantageusement, le dispositif de mesure 8 intègre au moins une sonde de température 13 pour mesurer la température du liquide 7, et au moins un autre capteur 15 de mesures pour mesurer la densité du liquide et/ou le débit gazeux.
En plus des capteurs de mesures de température 3 et de densité 15 du liquide, le dispositif de mesure 8 comporte au moins un autre capteur 151 de mesures pour réaliser des mesures mécaniques, électriques, optiques ou chimiques. Les mesures mécaniques comportent des mesures de pressions dans le liquide et/ou de débit gazeux et/ou de mouvement d'accélérations au sein du milieu liquide. Les mesures électriques comportent des mesures de tensions et/ou de courants à une ou plusieurs fréquences associées à des électrodes spécifiques disposées dans le dispositif de mesure 8, et/ou des mesures de fréquences de résonances d'oscillateurs intégrés dans le dispositif de mesure 8. Les mesures optiques comportent des mesures d'intensités lumineuses à une ou plusieurs longueur(s) d'ondes. Les mesures chimiques comportent des mesures de pH, des gaz dissouts dans le liquide, de potentiel Rédox, etc.

En outre, le dispositif de mesure 8 comporte un circuit électronique 17 relié à tous les capteurs et éléments électroniques et comprenant un microprocesseur 171 (ou microcontrôleur) configuré pour gérer l'acquisition de l'ensemble des capteurs et de réaliser des opérations de prétraitement : de filtrage, de moyennage, de comptage, de corrections, etc.

Le dispositif de mesure 8 comporte également un module de transmission ou de communication 19 pour transmettre au dispositif de contrôle 11 les données d'observation.

Dans le cas d'un dispositif de mesure 8 embarqué dans un flotteur 9 (Figs. 6, 7) le dispositif de mesure peut avantageusement comporter un module de gestion de l'énergie 21 comprenant une batterie pour alimenter tous les éléments électroniques du dispositif de mesure 8. En outre, le dispositif de mesure 8 peut également comporter des moyens qui peuvent être les mêmes capteurs de mesures électriques pour mesurer le niveau de flottaison du flotteur 9.

Selon un premier mode de réalisation, le microprocesseur 171 est configuré pour déterminer aux instants successifs des vecteurs de variables physiques d'intérêts relatives au processus biochimique en fonction des mesures correspondantes réalisées aux instants successifs. Ainsi, le module de transmission ou de communication 19 est configuré pour transmettre au dispositif de contrôle 11 les données d'observation comportant les vecteurs de variables physiques déterminés par le microprocesseur 171 ainsi que les mesures correspondantes.

Selon un autre mode de réalisation, les données d'observation transmises par le dispositif de mesure 8 au dispositif de contrôle 11 comportent seulement les mesures réalisées par les capteurs 13, 15, 151. Dans ce cas, le dispositif de contrôle 11 est configuré pour recevoir ces mesures et pour déterminer aux instants successifs les vecteurs de variables physiques d'intérêts relatives au processus biochimique en fonction des mesures correspondantes.

Chaque vecteur de variables physiques d'intérêts comporte une première variable de température ou de transfert de chaleur déterminée à partir de la différence de température entre deux points. Le vecteur de variables physiques d'intérêts comporte au moins une autre variable physique correspondant à la ou les mesure(s) autre que la température. Cette au moins une autre variable physique peut être une variable parmi les deuxième au douzième variables d'intérêts définies ci-dessus (voir également Figs. 6, 7).

La deuxième variable concerne le dégagement de gaz. Ceci peut être déterminé par un comptage local du nombre de bulles en utilisant un capteur de pression différentielle (tel que décrit dans la demande de brevet FR039275) qui pourrait avantageusement être intégré dans le flotteur 9 ou par des mesures de débit gazeux mesurant la quantité totale de gaz sortant de la cuve.

La troisième variable concerne la densité du liquide déterminée par des mesures de pression différentielle entre deux profondeurs en utilisant un capteur de pression (tel que décrit dans la demande de FR039275) qui pourrait avantageusement être intégré dans le flotteur 9 et/ou à partir des mesures de niveaux de flottaison du flotteur 9.

La quatrième variable concerne la conductivité électrique du liquide déterminée à partir des mesures électriques de tensions et/ou de courants à une ou plusieurs fréquences et/ou par des mesures de fréquence de résonance en utilisant un capteur électromagnétique qui pourrait avantageusement être intégré dans le flotteur 9.

La cinquième variable concerne la permittivité du liquide déterminée à partir des mesures électriques de préférence à plusieurs fréquences avec une électrode spécifique ou par des mesures de fréquence de résonance qui pourrait avantageusement être intégré dans le flotteur 9.

La sixième variable concerne le mouvement du liquide déterminé à partir des mesures d'accélération par l'intermédiaire d'un centrale inertielle ou accéléromètre qui pourrait avantageusement être intégré dans le flotteur 9.

La septième variable concerne la mesure du PH du liquide, déterminée à partir des mesures électriques avec une électrode spécifique qui pourrait avantageusement être intégré dans le flotteur 9.

La huitième variable concerne le potentiel redox déterminé à partir des mesures électriques avec une électrode spécifique qui pourrait avantageusement être intégrée dans le flotteur 9.

La neuvième variable concerne l'oxygène dissout déterminé à partir des mesures électriques et/ou mesures optiques.

La dixième variable concerne le $CO_2$ dissout déterminé à partir des mesures électriques avec une électrode spécifique et/ou à partir des mesures optiques qui pourraient avantageusement être intégrées dans le flotteur 9.

La onzième variable concerne le spectre d'absorption optique (turbidité, couleur) déterminé à partir des mesures optiques d'au moins deux longueurs d'ondes.

La douzième variable concerne le pouvoir rotatoire déterminé à partir des mesures optiques en utilisant une lumière polarisée qui varie par la présence de molécules chirales.

Le dispositif de contrôle 11 est configuré pour enregistrer les conditions initiales du processus biochimique par exemple, la composition du substrat liquide 7 dans la cuve 5 comme par exemple la concentration de l'ensemble des ingrédients comme par exemple le sucre, les caractéristiques du jus, les souches de levures, ou par des analyses chromatographiques etc. Le dispositif de contrôle 11 est configuré aussi pour récupérer les résultats du traitement, les enregistrer et les comparer à des seuils établis par l'utilisateur selon son expertise métier et les particularités du produit qu'il souhaite obtenir.

En outre, le dispositif de contrôle 11 est configuré pour commander des actionneurs 23 de régulation du processus biochimique. En effet, le dispositif de contrôle 11 est configuré pour commander la régulation du réacteur 3 biochimique par au moins une action $a_t$ à réaliser sur la cuve 5. A titre d'exemple, les actions peuvent comporter une modification de la vitesse d'agitation (circulation, rotation...) ou l'actionnement d'un brassage si le débit gazeux est trop faible ; une modification de la température (augmenter ou baisser la consigne de chauffage ou du circuit de refroidissement) par exemple, baisser la consigne de température si la variation de densité est trop forte ; une modification du débit d'apport en oxygène ; un apport de nutriments (sels minéraux, sucre, azote...) ou autres éléments d'activation ou de stabilisation du processus biochimique comme par exemple apport de l'oxygène ou de l'azote si le dégagement gazeux ralentit ; un apport de levures ou de souches bactériennes, etc.

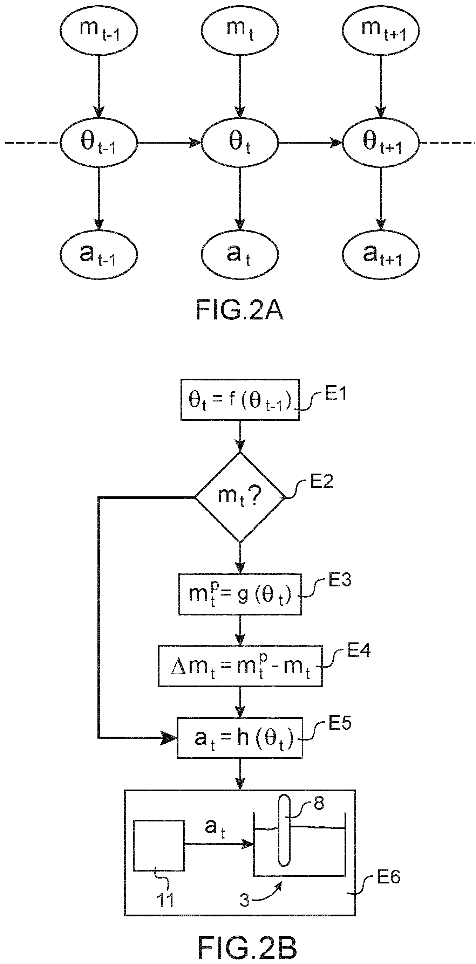

Les Figs. 2A et 2B illustrent un algorithme de contrôle du réacteur biochimique, selon un mode de réalisation préféré de l'invention. Plus particulièrement, la Fig. 2A représente l'algorithme de contrôle sous forme graphique et la Fig. 2B le représente sous forme d'un organigramme.

Cet algorithme repose sur la définition de trois fonctions temporelle définies sur une période T correspondant au processus biochimique. La première fonction *f* concerne la prédiction temporelle à un instant t, des valeurs des variables physiques $\theta_t$ en connaissance de leurs valeurs à un instant de temps précédent :
[Math. 1]

$$f: \theta_{t-1} \rightarrow \theta_t \qquad (1)$$

La deuxième fonction *g* associe les mesures $m_t$ réalisées par les capteurs aux variables physiques à un instant de temps t donné :
[Math. 2]

$$g: \theta_t \rightarrow m_t \qquad (2)$$

La troisième fonction *h* détermine l'action $a_t$ à réaliser dans la cuve selon les valeurs des variables physiques à un instant de temps t donné :
[Math. 3]

$$h: \theta_t \rightarrow a_t \qquad (3)$$

L'algorithme de contrôle est configuré pour déterminer pour chaque pas de temps t, une série d'actions $a_t$ en connaissance des variables physiques $\theta_t$ obtenues grâce aux mesures $m_t$ réalisées par les capteurs.

La Fig. 2A est un graphe orienté qui est organisé en forme matricielle à trois lignes et T colonnes et où chaque colonne représente un pas de temps t, allant de 1 à T. La première ligne correspond à une première séquence temporelle de nœuds représentatifs des mesures $m_t$, la deuxième ligne correspond à une deuxième séquence temporelle de nœuds représentatifs de vecteurs des variables physiques $\theta_t$ et la troisième ligne correspond à une troisième séquence temporelle de nœuds représentatifs de vecteurs d'actions $a_t$ de régulation. Les nœuds sur chaque colonne sont connectés par des flèches orientées de la première ligne à la deuxième ligne et ensuite de la deuxième ligne à la troisième ligne. En outre, les nœuds de la deuxième ligne (i.e. les vecteurs de variables physiques $\theta_t$) sont également connectés par des flèches orientées séquentiellement dans le sens croissant du temps de 1 à T.

Plus particulièrement, sur les flèches séquentielles de la deuxième ligne, le dispositif de contrôle 11 est configuré pour prédire aux instants successifs : d'une part des vecteurs prédictifs de variables physiques en fonction des vecteurs de variables physiques précédents issus des mesures et d'autre part, des mesures anticipatives en fonction des vecteurs prédictifs de variables physiques.

En outre, sur les flèches orientées de la première ligne vers la deuxième ligne, le dispositif de contrôle 11 est configuré pour calculer aux instants successifs des écarts de mesures entre les mesures anticipatives et les mesures réelles correspondantes. Le dispositif de contrôle 11 est configuré ensuite pour corriger aux instants successifs les vecteurs de variables physiques en fonction des écarts de mesures correspondants.

Sur la troisième ligne, le dispositif de contrôle 11 est configuré pour déterminer aux instants successifs des vecteurs d'actions de régulation en fonction des vecteurs de variables physiques correspondants. Ceci permet au dispositif de contrôle 11 de commander la régulation du réacteur 3 biochimique en déclenchant aux instants successifs des actions de régulation basées sur les vecteurs d'actions de régulation.

La Fig. 2B est un organigramme illustrant les étapes de contrôle du réacteur biochimique pour tout instant $t \in [1, T]$ au cours du processus biochimique selon le graphe orienté de la Fig. 2A.

A l'étape E1, le dispositif de contrôle est configuré pour réaliser une prédiction de chaque vecteur prédictif de variables physiques du processus biochimique en utilisant la première fonction temporelle *f* définissant des valeurs de variables physiques à l'instant t en connaissance de leurs valeurs à l'instant de temps précédent t-1 :
[Math. 4]

$$\theta_t = f(\theta_{t-1}) \quad (4)$$

Cette première fonction $f$ est prédéterminée par exemple, par un modèle de Markov standard d'ordre d'au moins 1.

L'étape E2 est un test pour vérifier si les capteurs sont fonctionnels à l'instant $t$, c'est-à-dire, si des mesures $m_t$ réalisées par les capteurs sont présentes à l'instant $t$. Si oui, alors on passe à l'étape E3, sinon, on va à l'étape E5.

L'étape E3 concerne la prédiction de chaque mesure anticipative en utilisant la deuxième fonction $g$ associant les mesures réalisées par le dispositif de mesure 8 à l'instant de temps t, au vecteur de variables physiques calculé à l'étape E1 :
[Math. 5]

$$m_t^p = g(\theta_t) \quad (5)$$

La deuxième fonction $g$ peut être prédéterminée par des équations liant les mesures réalisées par le dispositif de mesure 8 et les variables physiques.

L'étape E4 concerne une correction du vecteur de variables physiques $\theta_t$ par inversion (ou pseudo inversion) de la deuxième fonction $g$, et calcul de l'écart $\Delta m_t$ entre les vraies mesures $m_t$ faites par le flotteur et celles $m_t^p$ prédites : [Math. 6]

$$\Delta m_t = m_t^p - m_t \quad (6)$$

L'étape E5 concerne la détermination de chaque vecteur d'action de régulation en utilisant la troisième fonction $h$ définissant une correspondance à chaque instant de temps t, entre les actions à réaliser et les valeurs des variables physiques :
[Math. 7]

$$a_t = h(\theta_t) \quad (7)$$

La troisième fonction $h$ peut être prédéterminée par exemple, par expertise de l'utilisateur en sélectionnant de manière préalable des seuils sur les variables physiques de déclenchement d'actions.

A l'étape E6, le dispositif de contrôle 11 est configuré pour commander la régulation du réacteur 3 biochimique en déclenchant auxdits instants successifs des actions de régulation basées sur lesdits vecteurs d'actions de régulation.

Avantageusement, il est à noter qu'en cas de défaut de mesures, le dispositif de contrôle est configuré pour continuer à exécuter les étapes E1, E5 et E6 indépendamment des mesures. Ainsi, les vecteurs d'action de régulation peuvent être déterminés en fonction des vecteurs prédictifs de variables physiques en cas d'un défaut du dispositif de mesure 8.

Cette opération de contrôle peut prendre la forme d'un filtrage de Kalman si les fonctions $f$ et $g$ sont linéaires [Welch95], ou alors d'un filtrage stochastique type particulaire dans tout autre cas [Doucet09]. Avantageusement, elle ne nécessite pas de base de données préalable.

On notera que dans le cas où au moins une des trois fonctions est inconnue, elle peut être apprise automatiquement en connaissance d'au moins une série temporelle complète (i.e. t= 1 à T) de réaction biochimique associant des mesures $m_{1:T}$ et actions $\alpha_{1:T}$ réalisées dans la cuve 5.

En effet, une base de données peut être construite lors d'une phase d'apprentissage de régulation comportant plusieurs ensembles $\{m_{1:T}, \alpha_{1:T}\}$ de données de correspondance entre des mesures $m_{1:T}$ réalisées par le dispositif de mesure 8 et les actions $\alpha_{1:T}$ réalisées dans la cuve 5. Les données de correspondance sont générées automatiquement par un processus d'apprentissage. En effet, les variables physiques $\theta_{1:T}$ deviennent des variables cachées qui sont automatiquement estimées par le processus d'apprentissage, il n'est pas nécessaire de les connaitre tant que $m_{1:T}$ et $\alpha_{1:T}$ le sont. Les actions lors de la phase d'apprentissage de régulation du processus biochimique sont réalisées par un opérateur expert du métier.

Ainsi, au moins l'une quelconque des première, deuxième, et troisième fonctions peut être déterminée à partir des données de correspondance $\{m_{1:T}, \alpha_{1:T}\}$ acquises depuis la base de données. Cette détermination des fonctions peut être réalisée à l'aide d'un algorithme du type 'Expectation Maximisation (EM)' [Roweis99] s'il est supposé qu'elles soient linéaires.

Dans le cas où les fonctions ne sont pas linéaires, elles peuvent être déterminées à partir des données de correspondance $\{m_{1:T}, \alpha_{1:T}\}$ en utilisant un réseau de neurones récurrent (RNR) [Goodfellow16], ce même réseau servant lui-même d'algorithme de contrôle à la fin de son entrainement.

Une fois déterminées, les fonctions peuvent être utilisées pour le contrôle de toute réaction biochimique selon le schéma algorithmique précédemment expliqué.

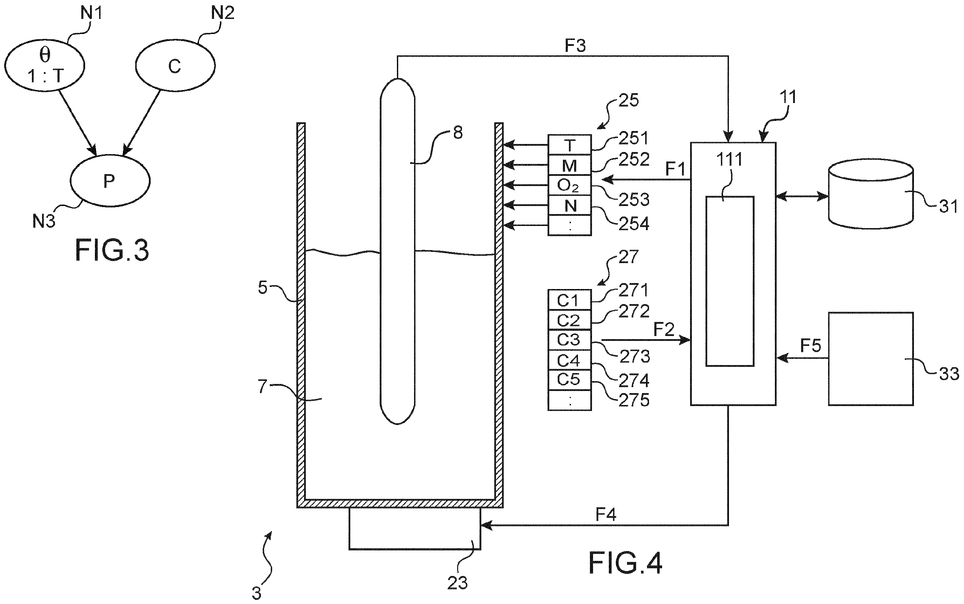

La Fig. 3 est un graphe orienté représentant un algorithme d'apprentissage de propriété du composé à la sortie du réacteur biochimique, selon un mode de réalisation préféré de l'invention.

Ce graphe illustre la construction par le dispositif de contrôle 11 d'un modèle d'apprentissage de propriété du composé en sortie du réacteur 3 biochimique. Le graphe comprend un premier nœud N1 représentant un vecteur de variables physiques $\theta_{1:T}$, un deuxième nœud N2 représentant un vecteur de caractéristiques $c$ des éléments mis en réaction dans la cuve 5, et un troisième nœud N3 représentant un vecteur de propriétés *p* du produit en sortie. Le modèle d'apprentissage de propriété permet de connecter chacun des premier $\theta_{1:T}$ et deuxième *c* nœuds au troisième nœud *p* par une flèche orientée.

En effet, à l'issue du processus biochimique, le composé obtenu possède différentes propriétés qui dépendent de l'évolution des vecteurs de variables physiques $\theta_{1:T}$ lors du traitement dans la cuve 5 ainsi que de certaines caractéristiques *c* des éléments mis en réaction dans la cuve 5.

Ces caractéristiques forment un vecteur de caractéristiques c comportant par exemple, la nature de microorganismes, des caractéristiques de la matière première, et éventuellement des nutriments additionnés. La nature de microorganismes comporte par exemple, le type de levures, de bactéries, de microalgues et leur quantité. Les caractéristiques de la matière première comportent par exemple, dans le cas du vin, la concentration de sucre, cépage, terroir, données météorologiques, de récoltes et de pressage. Dans d'autres applications, ces caractéristiques de matière première peuvent comporter la nature des eaux usées pour le traitement ou le substrat de culture bactériennes pour les bioréacteurs. Les nutriments additionnés comportent par exemple des activateurs de levures, sels minéraux, ou autres éléments de type charbon actif, glucose, etc.

Ainsi, le dispositif de contrôle 11 construit une base de données d'apprentissage de propriété comportant une collection d'ensembles $\{\theta_{1:T}, c\}$ entre les vecteurs de variables physiques $\theta_{1:T}$ et les vecteurs de caractéristiques *c* correspondantes.

Par ailleurs, les propriétés à l'issue du processus biochimique forment un vecteur de propriétés *p* révélateur de la qualité du composé. Celui-ci peut être évalué par différents appareils, comme ceux du type qui ne peuvent pas être intégrés pour une mesure in situ. A titre d'exemple, la concentration des différents métabolites peut être déterminée en laboratoire avec des équipements hors ligne (chromatographie, spectroscopie IR, etc.). La présence et concentration de microorganismes peut être réalisée par culture et analyses biologiques (PCR). D'autres analyses concernant par exemple, des observations organoleptiques (gouts, couleur, parfum, ..) du produit final qui peuvent être réalisées par expertise des spécialistes.

Le dispositif de contrôle 11 enregistre ainsi l'ensemble des observations et résultats des analyses en ligne ou hors ligne formant le produit final sous la forme de vecteurs de propriétés *p* du composé en sortie. Ainsi, après un ensemble de processus biochimiques, le modèle d'apprentissage de propriété comporte une collection d'ensembles de vecteurs de propriétés *p* du composé en sortie.

En outre, le dispositif de contrôle 11 est configuré pour construire des liens entre les vecteurs de variables physiques $\theta_{1:T}$ enregistrés à l'issue du processus de réaction biochimique et les vecteurs de caractéristiques *c* correspondants permettant d'obtenir les vecteurs de propriétés *p* du composé en sortie. Ces liens entre les vecteurs de variables physiques et les vecteurs de caractéristiques correspondants sont déterminés par l'estimation d'une fonction d'apprentissage *z* :
[Math. 8]

$$z: \theta_{1:T} \times c \rightarrow p \qquad (8)$$

En connaissance d'au moins un ensemble $\{\theta_{1:T}, c\}$ de la collection d'ensembles comprises dans la base de données, la fonction d'apprentissage *z* peut être estimée à l'aide de différents modèles statistiques de type régression polynomiale, ou modèle à noyau, ou réseau de neurones, etc. [Bishop06]. L'estimation de la fonction d'apprentissage *z* sera d'autant meilleure que la base de données est grande.

La fonction d'apprentissage *z* permet de comprendre l'influence des caractéristiques des éléments mis en réaction ou de l'évolution du processus biochimique dans les propriétés obtenues sur le composé. Ainsi, il devient possible d'adapter les caractéristiques des éléments mis dans la cuve 5 et de réguler l'évolution des variables physiques de réactions biochimiques par l'algorithme de contrôle afin d'obtenir des propriétés spécifiques du composé en sortie du réacteur 3. En effet, en connaissant le lien entre les variables physiques $\theta_{1:T}$ et les caractéristiques c qui donnent les propriétés *p*, le dispositif de contrôle peut spécifier lors du processus biochimique une série d'actions $\alpha_{1:T}$ pour « contraindre » les variables physiques $\theta_{1:T}$ à suivre un profil temporel ce qui en sortie ferait que le produit ait les propriétés souhaitées.

La Fig. 4 illustre très schématiquement un système et un procédé de surveillance in-situ d'un processus biochimique de fermentation dans un réacteur, selon un mode de réalisation de l'invention.

Plus particulièrement, la Fig. 4 est un exemple d'application du procédé de surveillance sur un processus de fermentation dans le domaine du vin, selon l'invention.

Le réacteur 3 biochimique comporte une cuve 5 destinée à recevoir un jus 7 sur lequel doit être réalisé le processus de fermentation pour fabriquer le vin. Comme dans la Fig. 1, le système de surveillance comporte un dispositif de mesure 8 (qui peut être intégré dans un flotteur) et un dispositif de contrôle 11.

Le dispositif de mesure 8 est configuré pour rester partiellement immergé du jus 7 contenu dans la cuve 5 tout en réalisant à des instants successifs des mesures de grandeurs physiques et plus particulièrement, des mesures de températures, de pressions, d'accélérations, et de fréquences.

Avantageusement, le dispositif de mesure 8 est configuré pour traiter les données de mesures afin de calculer des variables physiques d'intérêt comme par exemple, la densité, le débit de $CO_2$, la conductivité, et le mouvement du liquide, avant de transmettre au dispositif de contrôle 11 des données d'observation comprenant les variables physiques et les mesures correspondantes.

En variante, le dispositif de mesure 8 peut être configuré pour transmettre au dispositif de contrôle 11 des données d'observation comprenant uniquement les mesures correspondantes. Dans ce cas, c'est le dispositif de contrôle 11 qui traite les données de mesures pour calculer les variables physiques d'intérêt. Dans les deux cas, les variables physiques forment des indicateurs d'intérêt qui vont permettre au dispositif de contrôle 11 de réaliser automatiquement une rétroaction directe sur le processus de fermentation.

Le dispositif de contrôle 11 permet de saisir par l'intermédiaire d'un IHM 111 la recette 25 à appliquer au procédé. La recette peut comporter le profil de température 251, les conditions pour actionner le brassage 252, les conditions d'oxygénation 253, les conditions pour actionner l'apport de nutriments 254, etc.

La recette 25 comporte un ensemble de paramètres de seuils et de coefficients combinés de manière logique pour réaliser des consignes ou actions, comme dans les exemples non limitatifs ci-dessous.

Selon un premier exemple : Si (Densité < Seuil x) ou si (Débit gazeux < Seuil y) alors : Action de brassage selon une vitesse de brassage = Coef b * (Seuil y - Débit gazeux).

Selon un deuxième exemple : Si (Densité > Seuil w) et si (débit gazeux < Seuil z) alors : Ajout d'oxygène selon un Débit d'oxygène = Coef o *(Seuil z - Débit gazeux).

Selon un troisième exemple : Consigne température = Densité * Coef k + seuil I. En outre, le dispositif de contrôle 11 permet de saisir des caractéristiques d'entrées 27 du jus 7 qui peuvent être issues d'analyses complémentaires (en ligne ou hors ligne) ou de l'expertise du vigneron. Ces caractéristiques d'entrées comportent par exemple, le cépage 271, le terroir 272, la recette œnologique 273, le millésime 274, la souche de levure 275, etc. On notera que les caractéristiques sont des données d'entrée pour déterminer la recette 25 à appliquer au procédé sur la base d'expertise métier.

Par ailleurs, si nécessaire certaines mesures peuvent être réalisées hors ligne de façon ponctuelles et saisies par l'utilisateur ou transmis directement au dispositif de contrôle.

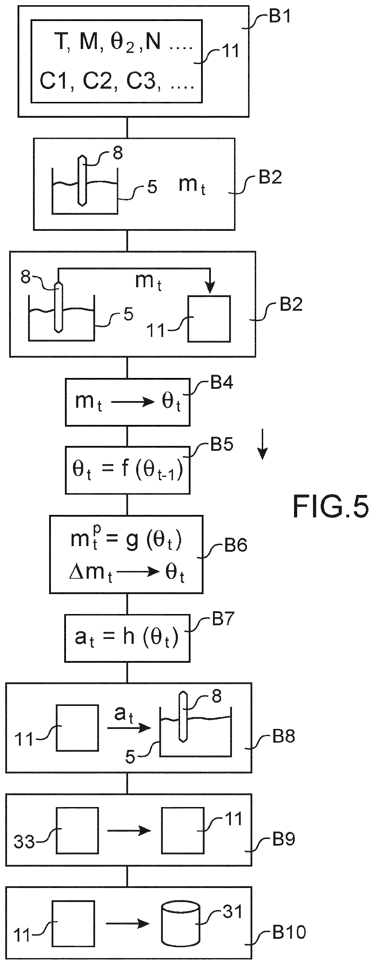

La Fig. 5 illustre très schématiquement un procédé de surveillance in-situ du processus biochimique de fermentation en relation avec le système de la Fig. 4.

Le bloc B1 concerne l'initialisation durant laquelle, le dispositif de contrôle 11 acquiert la recette à appliquer (flèche F1) au processus de fermentation. Cette recette peut être saisie par l'opérateur ou déterminée automatiquement à partir des caractéristiques d'entrées 27 (flèche F2) concernant le cépage 271, le terroir 272, la recette œnologique 273, le millésime 274, et la souche de levure 275.

Au bloc B2, le dispositif de mesure 8 réalise à chaque pas de temps t, des mesures de températures, de pressions, d'accélérations, de fréquences, etc.

Au bloc B3, le dispositif de mesure 8 transmet à chaque pas de temps t, au dispositif de contrôle 11 (flèche F3) des données d'observation comprenant les mesures $m_t$ réalisées. Eventuellement, le dispositif de contrôle 11 est également configuré pour acquérir d'autres mesures réalisées par exemple hors ligne de façon ponctuelles.

Au bloc B4, le dispositif de contrôle 11 calcule des variables physiques $\theta_t$ à partir des données de mesures $m_t$ correspondantes formant ainsi des vecteurs de variables physiques. Un vecteur de variables physiques peut comporter la température (ou transfert de chaleur), la densité, l'agitation, le débit de $CO_2$, l'acidité, la concentration en sucre, la conductivité, etc. Ces paramètres d'intérêt permettent au dispositif de contrôle 11 de déterminer une rétroaction immédiate en commandant (flèche F4) les actionneurs 23 de régulation du processus biochimique.

Aux blocs B5-B8, le dispositif de contrôle 11 réalise à chaque pas de temps t, les étapes décrites en relation avec la Fig 2B.

Au bloc B5, le dispositif de contrôle 11 prédit un vecteur prédictif de variables physiques en utilisant la première fonction f selon l'équation (4).

Au bloc B6, le dispositif de contrôle 11 prédit des mesures anticipatives en utilisant la deuxième fonction g selon l'équation (5), calcule les écarts entre les vraies mesures faites par dispositif de mesure 8 et celles prédites selon l'équation (6) et les utilise pour corriger le vecteur de variables physiques $\theta_t$.

Au bloc B7, le dispositif de contrôle 11 calcule le vecteur d'action de régulation en utilisant la troisième fonction h, selon l'équation (7) et affiche les valeurs des paramètres utiles à la régulation.

Au bloc B8, le dispositif de contrôle 11 commande la régulation du processus de fermentation en déclenchant des actions de régulation basées sur le vecteur d'action de régulation et sur la recette et consignes propres au processus.

Ces actions permettent d'agir immédiatement sur le processus de fermentation pour réguler des paramètres selon la recette et/ou pour prévenir et éviter des anomalies du type 'arrêt de fermentation' lorsque les levures ou bactéries n'agissent plus.

Un premier exemple d'action de régulation peut être une augmentation ou une diminution de la température du jus 7 selon le profil de température 251 spécifié au départ. En effet, le développement des bactéries dépend principalement de la température et ceci agit entre autres sur les arômes du vin. Ainsi, le profil de température est défini au départ par l'œnologue ou expert en vin selon le type du vin et l'arôme recherché.

Une deuxième action de régulation peut être le brassage 252 et vitesse de brassage selon la densité et/ou le débit gazeux. Une troisième action de régulation peut être l'oxygénation 253 et la quantité d'oxygénation selon le débit gazeux. Une quatrième action peut être l'ajout de levure 254 selon la température, et/ou la densité, et/ou le débit gazeux, etc.

Par ailleurs, le dispositif de contrôle 11 est avantageusement configuré pour créer des bases de données 31 de sortie qui permettront un apprentissage algorithmique sur la base d'appréciations organoleptiques comme l'évaluation de la qualité du produit au regard du profil aromatique recherché.

En effet, au bloc B9, à l'issue du processus de fermentation, le dispositif de contrôle 11 enregistre (flèche F5) les propriétés 33 du produit final (par exemple, l'évaluation et l'annotation du produit final faites par l'œnologue).

Au bloc B10, le dispositif de contrôle 11 constitue une base de données 31 d'apprentissage de propriété du produit final regroupant les caractéristiques du liquide au début du processus, les paramètres de la recette, l'enregistrement des mesures et variables physiques correspondantes, les analyses complémentaires (en ligne ou hors ligne), et des évaluations et propriétés P de la qualité du produit final.

Ainsi, en réalisant des enregistrements à l'issue de chaque nouveau processus de fermentation, la base de données 31 d'apprentissage de propriété comporte alors une collection de variables physiques $\theta_{1:T}$, de caractéristiques $c$ correspondantes, et des évaluations et propriétés $p$ du produit final à la sortie de chaque processus. Ceci permet d'automatiser le retour d'expérience en déterminant à l'aide de différents modèles statistiques une corrélation ou une fonction d'apprentissage $z$ associant les propriétés $p$ du produit final en fonction des variables physiques $\theta_{1:T}$ et des caractéristiques $c$ correspondantes, selon l'équation (8). Ceci permet d'optimiser tous les paramètres de pilotage du processus par exemple, les seuils et coefficients de la recette afin d'obtenir les meilleurs résultats.

La Fig. 6 illustre très schématiquement un système 1 de surveillance in-situ d'un processus biochimique dans un réacteur 3 biochimique, selon un mode de réalisation préféré de l'invention.

Ce mode de réalisation est similaire à celui de la Fig. 1 à l'exception du fait que le dispositif de mesure 8 est avantageusement intégré dans un flotteur 9 destiné à être introduit en flottant de manière partiellement immergée dans le liquide 7 contenu dans la cuve 5. Le flotteur 9 permet de pallier à toute variabilité de niveaux de remplissage de la cuve 5. Ce mode de réalisation est avantageux dans les cas de cuves 5 de grande taille comme par exemple pour la fabrication de boissons fermentées.

La Fig. 7 illustre très schématiquement un flotteur instrumenté, selon un mode de réalisation préféré de l'invention.

Le flotteur 9 d'une masse prédéterminée selon la gamme de mesure souhaitée est muni d'une tige 91 s'étendant vers le haut et d'un plongeur 92 destiné à le faire immerger partiellement et à assurer sa verticalité dans le liquide 7 lorsqu'il est introduit dans la cuve 5.

Le dispositif de mesure est integré dans le flotteur 9 de sorte que ce dernier comporte un circuit électronique 17, une ou plusieurs sondes de température 13a, 13b, des capteurs 15 de mesures mécaniques, électriques, et éventuellement optiques ainsi qu'un module de communication 19, et un module de gestion de l'énergie 21.

Les capteurs de mesures mécaniques comportent un capteur de pression différentielle 151 et un capteur de mouvement 152. Les capteurs de mesures électriques comportent au moins deux capteurs électromagnétiques 153a, 153b et éventuellement des capteurs 154 de mesure de pH, de mesures des gaz dissouts dans le liquide, de mesures du potentiel Rédox, etc. Des éventuels capteurs 155 de mesures optiques peuvent comporter des capteurs de mesures d'intensités lumineuses.

Le circuit électronique 17 est reliés à tous les capteurs 13, 15 et éléments électroniques 17, 21 intégrés dans le flotteur 9 et comporte un convertisseur analogique-numérique, une mémoire, et un microcontrôleur ou microprocesseur 171 destiné à gérer l'acquisition de l'ensemble des capteurs.

Les sondes de température 13a, 13b intégrées dans le flotteur 9 sont avantageusement déportées en haut et en bas de la partie large du flotteur 9 pour mesurer un éventuel gradient de température.

Le capteur de pression différentielle 151 est disposé sur la partie basse du flotteur 9 et comprend deux tubes 151a, 151b reliés de longueurs différentes ainsi qu'une membrane piézoélectrique (non illustré) qui se déforme sous l'effet d'une différence de pression générant une tension différentielle. Les extrémités libres des deux tubes 151a, 151b sont configurées pour être plongés dans le liquide 7 à deux profondeurs distinctes créant ainsi dans le circuit du capteur 151 de pression, une tension différentielle représentative de la pression différentielle entre les deux profondeurs distinctes. Le microprocesseur 171 du circuit électronique 17 est configuré pour déterminer la densité du liquide 7 en fonction de la pression différentielle.

En outre, la génération de bulles de gaz au niveau des extrémités des tubes 151a, 151b influence la mesure de la pression différentielle. Plus particulièrement, lorsque les bulles se décrochent, le capteur 151 génère des pics de tension représentatifs de l'apparition de bulles de sorte que chaque pic coïncide avec l'apparition d'une bulle de gaz au niveau de l'extrémité d'un des deux tubes 151a, 151b. Le microprocesseur 171 du circuit électronique 17 est configuré pour compter le nombre de bulles afin de déterminer le débit du gaz (en général du $CO_2$). Ceci est exposé en détail dans la demande de brevet de la demanderesse FR3039275.

Ainsi, le capteur de pression différentielle 151 permet de quantifier le dégagement gazeux issu de la réaction dans la cuve 5 et de déterminer la densité du liquide 7. Toutefois, au début, lorsqu'on place le flotteur 9 dans la cuve 5, une partie du liquide remonte dans les tubes 151a, 151b avant qu'ils se remplissent de gaz et par conséquent, durant ce régime transitoire on ne peut pas avoir une mesure précise de la densité. En revanche, en régime permanent, les tubes 151a, 151b sont remplis de gaz et par conséquent, le capteur 151 de pression différentielle délivre une mesure très précise de la densité du liquide 7.

Avantageusement, les capteurs électromagnétiques 153a, 153b ne sont pas affectés par ce phénomène et délivrent une mesure précise de la densité à la fois en régime transitoire et en régie permanent. Ainsi, le flotteur 9 comporte au moins des premier 153a et deuxième 153b capteurs électromagnétiques couplés au circuit électronique 17. Chaque capteur électromagnétique 153a, 153b comporte un circuit inductif-capacitif LC établissant un résonateur ayant une fréquence de résonance propre qui dépend de l'environnement fluidique du résonateur.

Plus particulièrement, chacun des capteurs 153a, 153b comporte un résonateur qui comprend une réactance active en forme d'électrode(s), dite électrode(s) de réactance associée à un élément de couplage passif correspondant. L'électrode de réactance peut être de nature capacitive ou inductive. En particulier, si cette électrode est de nature capacitive, alors l'élément de couplage correspondant est de nature inductive et vice versa, formant ainsi un résonateur dont la fréquence de résonance varie selon le niveau de flottaison du flotteur 9 et la nature du liquide 7 contenu dans la cuve 5.

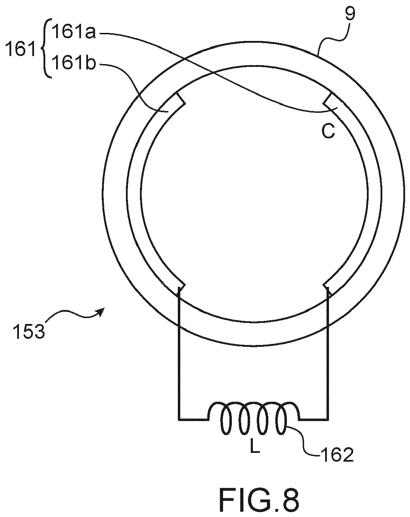

La Fig. 8 illustre de manière très schématique la disposition d'un capteur électromagnétique capacitif sur la surface latérale du flotteur, selon le mode de réalisation de la Fig. 7.

A titre d'exemple, on considère le cas d'un capteur électromagnétique 153 munis d'une électrode de réactance capacitive. L'électrode 161 de réactance comporte alors deux armatures 161a et 161b conductrices disposées l'une en face de l'autre sur la surface interne du flotteur 9 en épousant la forme cylindrique de cette surface. Ainsi, un condensateur de forme tubulaire est formé par les deux armatures 161a et 161b. Etant donné que la géométrie du condensateur est invariante, sa capacité C dépend essentiellement de la nature et niveau du liquide 7 contenu dans la cuve 5. On notera qu'en variante, les armatures 161a et 161b conductrices du capteur peuvent etre interdigitées.

En outre, les armatures 161a et 161b du condensateur de capacité C sont connectées à une inductance pouvant être une bobine 162 d'inductance L prédéterminée, formant ainsi un circuit de résonance dont la fréquence dépend de la capacité C qui peut être variable et de l'inductance L qui est constante. Le capteur électromagnétique 153 mesure la fréquence de résonance permettant ainsi au microprocesseur 171 de déduire la valeur de la capacité C et par conséquent, la permittivité du liquide au niveau des armatures 161a et 161b. Ainsi, la fréquence de résonance varie selon le niveau (i.e. interface liquide/air) du liquide 7 dans la cuve 5 et sa nature. La mesure par un capteur électromagnétique 153 de cette fréquence de résonance renseigne ainsi sur la nature et la configuration de cet environnement.

Le premier capteur électromagnétique 153a est intégré dans le plongeur de sorte que lorsque le flotteur 9 est introduit dans le liquide 7, l'électrode active du premier capteur électromagnétique 153a se trouve dans la partie totalement immergée. Ainsi, il n'y a pas d'interface air-liquide au niveau des armatures 161 et par conséquent, la fréquence de résonance mesurée est indicative uniquement de la nature du liquide 7. Le premier capteur électromagnétique 153a est ainsi configuré pour effectuer une mesure de référence relative aux propriétés physiques du liquide 7 et plus particulièrement, aux propriétés électriques telles que la conductivité et la permittivité du liquide 7 contenu dans la cuve 5.

Le deuxième capteur électromagnétique 153b est intégré dans la tige 91 du flotteur 9. En particulier, l'électrode active du deuxième capteur 153b est disposée sur toute la longueur de la tige 91. Ainsi, lorsque le flotteur 9 est introduit dans le liquide 7, l'électrode active comporte toujours une partie partiellement immergée dans le liquide. Le deuxième capteur 153b mesure alors le niveau d'immersion de la tige 91 et donc du volume du liquide déplacé indicatif de la densité du liquide 7. En effet, la variation des fréquences de résonance est indicative de la variation de la permittivité de l'isolant entre les armatures à cause du déplacement de l'interface air-liquide. On notera que la permittivité relative d'un liquide par rapport à l'air est assez significative. En particulier, l'introduction du flotteur 9 dans le liquide 7 fait augmenter le niveau du liquide qui par conséquent, fait augmenter la capacité générant une baisse de la fréquence de résonance.

Le microprocesseur 171 est configuré pour calculer la densité du liquide 7 sur la base de la mesure du volume du liquide déplacé réalisée par le deuxième capteur 153b et de la mesure des propriétés électriques du liquide réalisée par le premier capteur 153a.

En effet, en connaissant la masse du flotteur 9, le microprocesseur 171 peut estimer la valeur de la densité du liquide 7 à partir de la variation de niveau du liquide qui est proportionnelle au volume du liquide déplacé par le flotteur 9. En outre, la mesure des propriétés électriques du liquide 7 réalisée par le premier capteur électromagnétique 153a est une mesure de référence qui permet au microprocesseur 171 de corriger la première estimation et de déterminer la densité du liquide 7 avec une plus grande précision.

Ainsi, les capteurs électromagnétiques 153a, 153b donnent une mesure précise de la densité du liquide 7 qui permet de contourner l'imprécision de mesure du capteur de pression différentielle 151 au début de la réaction. En plus, le fait d'avoir deux mesures indépendantes de densité permet de renforcer la précision de la mesure en régime permanent et éventuellement de détecter tout dysfonctionnement des capteurs.

Le capteur de mouvement 152 est par exemple, une centrale inertielle ou un accéléromètre à trois axes qui peut être intégré dans la partie immergée 92 du flotteur 9. Ce capteur de mouvement 152 est configuré pour mesurer le mouvement du flotteur 9 et indirectement celle du liquide 7 et pour fournir cette information indirecte sur les mouvements du liquide au microprocesseur 171. En effet, le liquide 7 peut être soumis à un mouvement de brassage contrôlé. Le microprocesseur 171 récupère les mesures fournies par le capteur de mouvement 152 pour calculer le mouvement du liquide 7. La mesure des mouvements du liquide 7 lorsqu'il est soumis à une agitation contrôlée, permet d'estimer sa viscosité. L'estimation de la viscosité pourrait également être envisagée soit en appliquant une rotation (par exemple avec un agitateur) ou en écartant le flotteur 9 de sa position d'équilibre et en mesurant le temps nécessaire pour y revenir.

Avantageusement, le capteur de mouvement 152 est en outre configuré pour valider ou corriger l'acquisition selon si le liquide 7 est au repos ou en mouvement. Il permet également d'éliminer les artefacts liés aux perturbations extérieures.

Le flotteur 9 peut également comporter d'autres capteurs 154 adaptés pour mesurer des propriétés chimiques du liquide. A titre d'exemple, le flotteur 9 peut comporter un capteur de mesure de pH, des capteurs de mesures des gaz ($O_2$, $CO_2$) dissouts dans le liquide, un capteur de mesure du potentiel Rédox, un capteur de mesure de concentration de molécules spécifique par la technique d'électrode sélective d'ions ISE (ion selective electrode), etc. Avantageusement, les capteurs 154 de mesures de propriétés chimiques peuvent être intégrés dans le flotteur 9 en utilisant des électrodes en DLC (diamon like carbon) qui permet de miniaturiser ces capteurs. Leur sélection et leur usage dépendront des besoins de l'application et de l'agressivité du milieu.

On notera que la fréquence des acquisitions de chaque capteur peut être définie selon les spécificités de chaque mesure (par exemple : supérieur à 1Hz pour la mesure de pression différentielle, inférieur à 0.1Hz pour la mesure de densité par la méthode électromagnétique, etc.).

Le module de communication 19 sans fils comporte une antenne 20 intégrée dans la tige 91 du flotteur 9 et connectée aux différents capteurs 13, 15 par l'intermédiaire du circuit électronique 17. L'antenne 20 est configurée pour transmettre les données relatives aux mesures réalisées par les capteurs au dispositif de contrôle 11 selon un système de communication sans fils (par exemple : NFC, RFID, Blue tough, Wifi...) à basse énergie. Ainsi, les données sont communiquées par la partie émergente du flotteur 9 au dispositif de contrôle 11 pour qu'il puisse commander les différentes actions à mener pour la régulation du processus biochimique.

Le module de gestion de l'énergie 21 comporte une batterie ou un autre moyen d'alimentation avantageusement intégré dans le plongeur 92 du flotteur 9. Selon l'usage, l'autonomie nécessaire pour la durée minimale d'une acquisition permettra de dimensionner le moyen d'alimentation. Dans le cas où l'alimentation électrique est une batterie, cette dernière est disposée de préférence en partie basse pour servir de lest. En contraste, une télé alimentation serait située de préférence dans la partie non immergée.

Avantageusement, le système 1 de surveillance comporte un module 31 de calibration et de recharge inductive destinés à calibrer le flotteur 9 et à télé-recharger la batterie intégrée dans le flotteur 9. On notera que selon la nature de la cuve 5 et du liquide 7, le flotteur 9 peut être télé-alimenté de manière continue. Le module 31 de calibration et de recharge inductive peut être avantageusement compris dans un étui 33 de protection du flotteur 9.

Le microprocesseur 171 du circuit électronique 17 en relation avec le convertisseur analogique-numérique et la mémoire est configuré pour gérer l'acquisition de l'ensemble des capteurs 13, 15 et pour réaliser un prétraitement : de filtrage, moyennage, comptage et corrections permettant ainsi de ne transmettre que la partie utile de ces informations. Par exemple, le microprocesseur 171 peut appliquer des facteurs correctifs de mesures par exemple, de température et de pression en ne tenant pas compte des mesures en cas d'agitation, ou en appliquant des facteurs correctifs aux valeurs mesurées si les capteurs sont sensibles aux conditions de températures et pressions.

En outre, le microprocesseur 171 est configuré pour fusionner toutes les données de mesures afin de détecter toute anomalie provenant par exemple, d'une détérioration ou d'encrassement d'un capteur. Il est configuré pour émettre une alerte pour maintenance préventive. Le microprocesseur 171 est adapté aussi pour mesurer la réponse du système lorsque le liquide est soumis à une action extérieure : agitation, modification de la consigne en température, apport de nutriments, de levures, oxygénation, etc.

Le microprocesseur 171 est configuré aussi pour réaliser un traitement des mesures afin d'extraire les variables physiques caractérisant le liquide 7 qui sont nécessaires au pilotage du processus biochimique dans la cuve 5.

Ainsi, le circuit électronique 7 permet de gérer l'acquisition de l'ensemble des capteurs, d'extraire les données utiles et de les mettre en forme avant de les transmettre vers le dispositif de contrôle 11.

Bien entendu, diverses modifications peuvent être apportées par l'homme du métier à l'invention qui vient d'être décrite, uniquement à titre d'exemples non limitatifs.

**Bibliographies**

[Welch95]: An introduction to the Kalman filter, G. Welch, G. Bishop, technical report 2006.

[Doucet09]: A Tutorial on Particle Filtering and Smoothing: Fifteen years later, A. Doucet, A. M. Johansen, technical report 2012.

[Roweis99]: A unifying review of linear Gaussian models, S. Roweis, Z. Ghahramani, Neural Computation 1999.

[Goodfellow16]: Deep learning, I. Goodfellow, Y. Bengio, A. Courville, Y Bengio, MIT press 2016.

[Bishop06]: Pattern recognition and machine learning, C. M. Bishop, Springer 2006.

**Revendications**

1. Système de surveillance in-situ d'un processus biochimique dans un réacteur comprenant une cuve (5) destinée à recevoir un liquide (7), **caractérisé en ce qu'**il comporte :

   - un dispositif de mesure (8) destiné à être introduit dans ladite cuve (5), ledit dispositif de mesure (8) étant instrumenté de capteurs configurés pour réaliser à des instants successifs des mesures relatives au processus biochimique et pour transmettre auxdits instants successifs des données d'observation représentatives d'au moins la température et la densité du liquide, et
   - un dispositif de contrôle (11) configuré pour commander auxdits instants successifs la régulation du réacteur (3) biochimique en fonction desdites données d'observation reçues depuis le dispositif de mesure (8).

2. Système selon la revendication 1, **caractérisé en ce que** lesdites mesures comportent des mesures de température et de densité du liquide et au moins un autre type de mesures parmi les mesures suivantes : mesures mécaniques de pressions et/ou de débit gazeux et/ou d'accélérations, et/ou de niveau de flottaison, mesures électriques de tensions et/ou de courants et/ou de fréquences de résonance, et mesures optiques.

3. Système selon la revendication 1 ou 2, **caractérisé en ce que** le dispositif de mesure comporte un microprocesseur (171) configuré pour déterminer auxdits instants successifs des vecteurs de variables physiques relatives au processus biochimique en fonction des mesures correspondantes réalisées auxdits instants successifs, les données d'observation transmises par le dispositif de mesure (8) audit dispositif de contrôle (11) comportant lesdits vecteurs de variables physiques et lesdites mesures correspondantes.

4. Système selon la revendication 1 ou 2, **caractérisé en ce que** les données d'observation transmises par le dispositif de mesure audit dispositif de contrôle (11) comportent lesdites mesures et **en ce que** le dispositif de contrôle est configuré pour déterminer auxdits instants successifs des vecteurs de variables physiques relatives au processus biochimique en fonction des mesures correspondantes.

5. Système selon la revendication 3 ou 4, **caractérisé en ce que** chacun desdits vecteurs de variables physiques comporte une variable de température, une variable de densité du liquide déterminée à partir des mesures de niveaux de flottaison et/ou de pressions et au moins une autre variable parmi les variables suivantes : dégagement gazeux, déterminée à partir des mesures de pressions, conductivité et/ou permittivité électriques du liquide déterminées à partir des mesures électriques, mouvement du liquide déterminé à partir des mesures d'accélération, PH et/ou potentiel redox déterminés à partir des mesures électriques, oxygène et/ou $CO_2$ dissout déterminés à partir des mesures électriques et/ou mesures optiques, spectre d'absorption optique et/ou pouvoir rotatoire déterminés à partir des mesures optiques.

6. Système selon l'une quelconque des revendications précédentes, **caractérisé en ce que** le dispositif de contrôle (11) est configuré pour commander la régulation du réacteur biochimique par au moins une action parmi les actions suivantes : modification de la vitesse d'agitation, modification de la température, modification du débit d'apport en oxygène, apport de nutriments ou autres éléments d'activation ou de stabilisation du processus biochimique, apport de levures ou de souches bactériennes.

7. Système selon l'une quelconque des revendications 4 à 6, **caractérisé en ce que** le dispositif de contrôle (11) est configuré pour :

   - prédire auxdits instants successifs des vecteurs prédictifs de variables physiques en fonction desdits vecteurs de variables physiques précédents issus des mesures,
   - prédire auxdits instants successifs des mesures anticipatives en fonction desdits vecteurs prédictifs de variables physiques,
   - calculer auxdits instants successifs des écarts de mesures entre les mesures anticipatives et les mesures réelles correspondantes,
   - corriger auxdits instants successifs les vecteurs de variables physiques en fonction desdits écarts de mesures,
   - déterminer auxdits instants successifs des vecteurs d'actions de régulation en fonction desdits vecteurs de variables physiques correspondants, et
   - commander la régulation du réacteur biochimique en déclenchant auxdits instants successifs des actions de régulation basées sur lesdits vecteurs d'actions de régulation.

**8.** Système selon la revendication 7, **caractérisé en ce que** le dispositif de contrôle (11) est configuré pour déterminer les vecteurs d'actions de régulation en fonction des vecteurs prédictifs de variables physiques en cas de défaut de mesures par le dispositif de mesure (8).

**9.** Système selon la revendication 7 ou 8, **caractérisé en ce que** le dispositif de contrôle (11) est configuré pour

- prédire chaque vecteur prédictif de variables physiques en utilisant une première fonction f temporelle définissant des valeurs de variables physiques en connaissance de leurs valeurs à un instant de temps précédent, ladite première fonction f étant prédéterminée par un modèle de Markov standard d'ordre d'au moins 1,
- prédire chaque mesure anticipative en utilisant une deuxième fonction g associant les mesures réalisées par le flotteur aux variables physiques à un instant de temps donné, ladite deuxième fonction g étant prédéterminée par des équations liant les mesures réalisées par le dispositif de mesure et les variables physiques, et
- détermination de chaque vecteur d'actions de régulation en utilisant une troisième fonction h définissant une correspondance entre les actions à réaliser et les valeurs des variables physiques à un instant de temps donné, ladite troisième fonction h étant prédéterminée par une sélection préalable de seuils de déclenchement d'actions.

**10.** Système selon la revendication 9, **caractérisé en ce qu'**il comporte une base de données construite lors d'une phase d'apprentissage de régulation comportant des données de correspondance entre des mesures $m_{1:T}$ réalisées par le dispositif de mesure et des actions $a_{1:T}$ réalisées dans la cuve, lesdites données de correspondance étant générées automatiquement par un processus d'apprentissage, et **en ce qu'**au moins l'une quelconque des première, deuxième et troisième fonctions est déterminée à partir desdites données de correspondance acquises depuis ladite base de données.

**11.** Système selon l'une quelconque des revendications précédentes, **caractérisé en ce que** le dispositif de contrôle (11) est configuré en outre pour construire un modèle d'apprentissage de propriété définissant des liens associant des propriétés p du produit final en fonction des variables physiques $\theta_{1:T}$ et caractéristiques c correspondantes.

**12.** Système selon la revendication 11, **caractérisé en ce que** le dispositif de contrôle (11) est configuré pour déterminer lesdits liens par estimation d'une fonction d'apprentissage au moyen d'un modèle statistiques de type régression polynomiale, ou modèle à noyau, ou réseau de neurones.

**13.** Système selon l'une quelconque des revendications précédentes, **caractérisé en ce que** le dispositif de mesure comporte au moins une sonde (13a, 13b) de température, et un capteur de pression différentielle (151) comprenant deux tubes (151a, 151b) reliés de longueur différentes.

**14.** Système selon l'une quelconque des revendications précédentes, **caractérisé en ce que** le dispositif de mesure est intégré dans un flotteur (9) comportant :

- au moins une sonde (13a, 13b) de température,
- un capteur de pression différentielle (151) disposé sur la partie basse du flotteur (9) et comprenant deux tubes (151a, 151b) reliés de longueur différentes,
- au moins deux capteurs électromagnétiques (153a, 153b) comportant des armatures fixées sur les parois du flotteur,
- un capteur de mouvement (152) configuré pour mesurer les mouvements du liquide,
- un module de communication (19) sans fils comprenant une antenne (20) disposée sur la partie haute du flotteur, le module de communication (19) étant destiné à transmettre les données relatives aux mesures réalisées par les capteurs,
- un module de gestion de l'énergie (21) comprenant un moyen d'alimentation électrique disposé dans la partie basse du flotteur, et
- un circuit électronique (17) relié à tous les capteurs et éléments électroniques intégrés dans le flotteur, le circuit électronique comprenant un microprocesseur (171) destiné à gérer l'acquisition de l'ensemble des capteurs et la transmission des données.

**15.** Système selon la revendication 14, **caractérisé en ce qu'**il comporte un module (31) de calibration et de recharge inductive destiné à calibrer le flotteur (9) et à télé-recharger le moyen d'alimentation intégrée dans le flotteur, ledit module (31) de calibration et de recharge inductive étant compris dans un étui (33) de protection du flotteur.

**16.** Procédé de surveillance in-situ d'un processus biochimique dans un réacteur comprenant une cuve (5) destinée à recevoir un liquide (7), **caractérisé en ce qu'**il comporte les étapes suivantes :

- réaliser à des instants successifs des mesures relatives au processus biochimique,
- transmettre auxdits instants successifs des données d'observation représentatives d'au moins la température et la densité du liquide, et
- commander auxdits instants successifs la régulation du réacteur biochimique en fonction desdites données d'observation.

FIG.1

FIG.2A

FIG.2B

FIG.3

FIG.4

EP 4 019 622 A1

FIG.5

FIG.6

FIG.7

FIG.8

Europäisches
Patentamt

European
Patent Office

Office européen
des brevets

## RAPPORT DE RECHERCHE EUROPEENNE

Numéro de la demande

**EP 21 21 7333**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2019/300841 A1 (ARON KATHRYN L [US] ET AL) 3 octobre 2019 (2019-10-03) * revendications 1,2 * ----- | 1-16 | INV. C12M1/36 C12M1/09 C12M1/34 |
| A | EP 0 271 380 A1 (AGRONOMIQUE INST NAT RECH [FR]; CEMAGREF [FR]) 15 juin 1988 (1988-06-15) * revendication 1 * ----- | 1-16 | G01N33/14 G01N7/20 |
| A | FR 3 039 275 A1 (COMMISSARIAT ENERGIE ATOMIQUE [FR]) 27 janvier 2017 (2017-01-27) * revendications 1,2,5,6 * ----- | 1-16 | |
| A | WO 2006/086489 A1 (BROADLEY JAMES CORP [US]; WEST LARRY EUGENE [US]) 17 août 2006 (2006-08-17) * revendications 1,8,22 * ----- | 1-16 | |
| A | WO 2010/002745 A1 (SOLIX BIOFUELS INC [US]; UNIV COLORADO STATE RES FOUND [US] ET AL.) 7 janvier 2010 (2010-01-07) * revendications 1,6 * ----- | 1-16 | DOMAINES TECHNIQUES RECHERCHES (IPC) |
| A | EP 2 738 528 A1 (AIRBUS OPERATIONS GMBH [DE]) 4 juin 2014 (2014-06-04) * revendication 1 * ----- | 1-16 | C12M G01N G01F |

Le présent rapport a été établi pour toutes les revendications

| Lieu de la recherche | Date d'achèvement de la recherche | Examinateur |
|---|---|---|
| Berlin | 10 mai 2022 | Jones, Laura |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 21 21 7333

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-05-2022

| Document brevet cité au rapport de recherche | Date de publication | Membre(s) de la famille de brevet(s) | Date de publication |
|---|---|---|---|
| US 2019300841 A1 | 03-10-2019 | AUCUN | |
| EP 0271380 A1 | 15-06-1988 | DE 3787515 T2 | 17-02-1994 |
| | | EP 0271380 A1 | 15-06-1988 |
| | | ES 2043682 T3 | 01-01-1994 |
| | | FR 2606514 A1 | 13-05-1988 |
| FR 3039275 A1 | 27-01-2017 | AUCUN | |
| WO 2006086489 A1 | 17-08-2006 | EP 1851303 A1 | 07-11-2007 |
| | | US 2005158701 A1 | 21-07-2005 |
| | | US 2010099172 A1 | 22-04-2010 |
| | | US 2012065782 A1 | 15-03-2012 |
| | | US 2013245830 A1 | 19-09-2013 |
| | | US 2015010990 A1 | 08-01-2015 |
| | | US 2016348059 A1 | 01-12-2016 |
| | | WO 2006086489 A1 | 17-08-2006 |
| WO 2010002745 A1 | 07-01-2010 | AU 2009267214 A1 | 07-01-2010 |
| | | BR PI0914593 A2 | 15-12-2015 |
| | | CN 102131383 A | 20-07-2011 |
| | | US 2012107921 A1 | 03-05-2012 |
| | | WO 2010002745 A1 | 07-01-2010 |
| EP 2738528 A1 | 04-06-2014 | EP 2738528 A1 | 04-06-2014 |
| | | US 2014150549 A1 | 05-06-2014 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 039275

- FR 3039275

**Littérature non-brevet citée dans la description**

- **KALMAN FILTER ; G. WELCH ; G. BISHOP.** *technical report,* 2006
- **A. DOUCET ; A. M. JOHANSEN.** *technical report,* 2012
- **S. ROWEIS ; Z. GHAHRAMANI.** *Neural Computation,* 1999

- **I. GOODFELLOW ; Y. BENGIO ; A. COURVILLE ; Y BENGIO.** Deep learning. MIT press, 2016
- **C. M. BISHOP.** Pattern recognition and machine learning. Springer, 2006